# EUROPEAN PATENT APPLICATION

(11) **EP 0 780 393 A1**
(43) Date of publication of application: **25.06.1997**
(21) Application number: 95930705.9
(22) Date of filing: 06.09.1995
(51) Int. Cl.: C07D 501/59, A61K 31/545

(54) **NOVEL CRYSTAL OF CEPHALOSPORIN COMPOUND**

(30) Priority: 07.09.1994 ZA 9406888; 08.03.1995 US 400770
(71) Applicant: FUJISAWA PHARMACEUTICAL CO., LTD., Osaka-shi Osaka 541 (JP)
(72) Inventor: KAWABATA, Kohji, Hyogo 666-01 (JP); TERASAWA, Takeshi, Osaka 586 (JP); OHKI, Ayako, Takarazuka-shi Hyogo 665 (JP); SHIRAI, Fumiyuki, Osaka 532 (JP); YAMAMOTO, Hirofumi, Osaka 563 (JP); KAWAKAMI, Ryoichi, Osaka 560 (JP); HAMAGUCHI, Chiaki, Hyogo 651-12 (JP)
(74) Representative: Türk, Gille, Hrabal, Leifert
(86) International application number: JP9501769
(87) International publication number: WO9607659

(57) **Abstract**

A novel crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof.

Said compound is a cephalosporin antibiotic having very strong antibacterial activity which exhibits superior characteristics physically, chemically and pharmaceutically.

## Description

### Technical Field

This invention relates to a novel crystal of 7β-[2-(2-aminothiazol-4-yl)-2-(Z)-(hydroxyimino)acetamido]-3-[(pyrazol-4-yl)methylthio]-3-cephem-4-carboxylic acid [hereinafter referred to briefly as compound (I)] or a salt thereof having strong antibacterial activity which is shown in the following chemical formula.

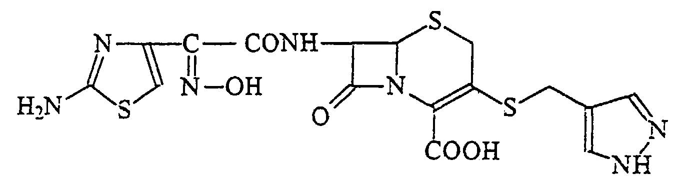

### Background Art

Compound (I) or a salt thereof is a cephalosporin antibiotic having very strong antibacterial activity and its potent antibacterial action and high urinary excretion in animals, which suggests that it can be of value as a cephalosporin for oral use. However, the noncrystalline form or Compound (I) or a salt thereof which was obtained until now is chemically and physically unstable so that it has been impossible to exploit the compound as an active pharmaceutical.

### Disclosure of the invention

The inventors of this invention succeeded, in obtaining Compound (I) or a salt thereof in a crystalline form. The inventors further found that compared with the conventional noncrystalline product, this crystalline product is by far more stable against heat and light and exhibits superior characteristics physically, chemically and pharmaceutically.

This invention is described in detail as follows.

The crystal of 7β-[2-(2-aminothiazol-4-yl)-2-(Z)-(hydroxyimino)acetamido]-3-[(pyrazol-4-yl)methylthio]-3-cephem-4-carboxylic acid or a salt thereof according in this invention means a substantially pure crystal of 7β-[2-(2-aminothiazol-4-yl)-2-(Z)-(hydroxyimino)acetamido]-3-[(pyrazol-4-yl)methylthio]-3-cephem-4-carboxylic acid or a salt thereof which does not contain other effective amount of pharmacologically active components, and which includes all kind of solvates and clathrates.

Such a crystal of solvate of compound (I) or a salt thereof may contain a solvent as forming a part of the crystal lattice, as included within the crystal lattice, as adsorbed on the lattice, or in any mixture of such varied forms.

The solvent to be contained in such a crystal can be any solvent that has no bad effect on human physiology, thus including water and various organic solvents, e.g. lower alcohols such as ethyl alcohol, isopropyl alcohol, etc. and lower alkyl ketones such as acetone, methyl ethyl ketone, and so on. These solvents can be used singly or in various combinations. Preferred is a solvate with water only or a mixed solvate with water and an organic solvent, particularly water and a lower alcohol such as ethyl alcohol or isopropyl alcohol. The crystal of Compound (I) or a salt thereof according to this invention is not morphologically restricted but includes solvates in which the solvent of crystal constitutes a part of the crystal structure or is included within the crystal structure of Compound (I) or a salt thereof such as clathrates. Unless otherwise noted in this specification, the term 'crystal of solvate' is used to cover not only clathrate forms of crystals but also crystals of solvate crystal stick a solvent. The amount of a solvent in the solvate crystal per molecule of Compound (I) or a salt thereof is not restricted but is preferably 0.1-6 molecules and, more preferably, 2-4 molecules. Furthermore, where the solvent of crystal comprises both water and an organic solvent, the amount of water is generally 0.1-6 molecules and preferably 0.1-4 molecules per molecule of Compound (I) or a salt thereof and that of the organic solvent is preferably 0.1-2 molecules. Most preferable amounts are 2-3 molecules for water and 0.1-2 molecules for the organic solvent. When water is the sole solvent, the amount of water per molecule of Compound (I) or a salt thereof is 1-6 molecules and preferably 1-4 molecules and, more preferablly 2-3 molecules.

The inventors of this invention further investigated the crystal of Compound (I) in regard to its powder X-ray diffraction pattern. As a result, we discovered that the preferred crystal of Compound (I) has a powder X-ray diffraction pattern with peaks at the diffraction angles of ca. 7.5°, ca. 11.3°, ca. 19.5°, ca. 21.3°, ca. 24.5°, ca. 25.9°, and ca. 29.0°. Crystals with powder X-ray diffraction pattern features substantially similar to the above also fall within the scope of this invention.

Suitable pharmaceutically acceptable salts of the object compound (I) are conventional non-toxic pharmaceutically acceptable salts and include a salt with a base or an acid addition salt, for example an inorganic base salt [a metal salt such as an alkali metal salt (e.g. sodium salt, potassium salt, etc.) and an alkaline earth metal salt (e.g. calcium salt, magnesium salt, etc.), an ammonium salt etc.], an organic base salt [e.g. trimethylamine salt, triethylamine salt, pyridine salt, picoline salt, dicyclohexylamine salt, N,N'-dibenzylethylenediamine salt, etc.], an organic acid salt [e.g. formate, acetate, trifluoroacetate, maleate, tartrate, methanesulfonate, benzenesulfonate, toluenesulfonate, etc.], an inorganic acid salt [e.g. hydrochloride, hydrobromide, sulfate, phosphate, etc.], a salt with an amino acid [e.g. arginine salt, aspartic acid salt, glutamic acid salt, etc.], and the like.

The process for producing the above-described crystal of Compound (I) or a salt thereof in accordance with this invention is now described in detail.

Non-crystalline form of Compound (I) or a salt thereof can be synthesized according to Preperations 1-21 of this specification. It should be understood that Compound (I) or a salt thereof as prepared by any other processes can likewise be employed in the following process for producing the crystal in this invention.

The crystal of Compound (I) or a salt thereof can be prepared by allowing crystals to separate out from a solution containing Compound (I) or a salt thereof under acidic conditions at cooled temperature, room temperature or elevated temperature.

A preferred example of said solution containing Compound (I) or a salt thereof is a solution of an alkali metal salt of Compound (I) or a salt thereof in water or a mixture of water and an organic solvent. The preferred organic solvent for use in the preparation of said solution includes lower alcohols such as ethyl alcohol, isopropyl alcohol, etc. and lower alkyl ketones such as acetone, methyl ethyl ketone and so on.

If desired, a solution containing Compound (I) or a salt thereof is chromatographed on a column of activated carbon, nonionic adsorbent resin, alumina, acidic aluminum oxide or the like and the eluate is then acidified with an acid at cooled temperature, room temperature or elevated temperature, optionally followed by concentration, to provide the objective crystal of Compound (I) or a salt thereof. The amount of the acid to be added is preferably just sufficient to bring the pH of the solution into the range of 1-5. The type of said acid is not critical and the usual reagent such as hydrochloric acid can be employed.

Among crystals of Compound (I) or a salt thereof that can be obtained in the foregoing manner, the crystal of solvate containing an organic solvent can be desolvated ,if desired. For the desolvation, the usual vacuum drying procedure may disrupt the crystal structure. Therefore, supercritical gas extraction using carbon dioxide gas or the like (JP Kokai H-1-238589) or the draft drying process using a moisture-laden gaseous medium can be used to efficiently remove the organic solvent.

In the draft drying process using a moisture-laden gaseous medium, moist air or nitrogen gas controlled at not less than 20% R.H., preferably not less than 30% R.H., is blown through the crystal and can remove the organic solvent a conventional manner. This process enables control of the water ratio in the crystals by selecting the humidity of gas.

By allowing the thus-obtained crystal of Compound (I) or a salt thereof to stand in an organic solvent vapor, the organic solvent in the crystal can be replaced with a different organic solvent or a different amount of the solvent. It is also possible to adjust the amount of water in the crystal by allowing the crystal to stand in the air containing a different amount of water.

The crystal of Compound (I) or a salt thereof obtained in accordance with this invention can be formulated with a pharmaceutically acceptable carrier suitable for oral, parenteral or external medication, such as an organic or inorganic excipient, which may be solid or liquid, and put to use in per se known dosage forms containing Compound (I) or a salt thereof as an active ingredient.

Such dosage forms can be provided in a variety of solid unit dosage forms such as tablets, granules, powders, capsules, etc. or liquid unit dosage forms such as suspensions.

Where necessary, such dosage forms can be supplemented with auxiliary agents, stabilizers, wetting agents and other conventional additives such as lactose, citric acid, tartaric acid, stearic acid, magnesium stearate, terra alba, sucrose, corn starch, talc, gelatin, agar, pectin, peanut oil, olive oil, cacao butter, and ethylene glycol, among others.

The dosage of the crystal of Compound (I) or a salt thereof is dependent on the patient's age and other factors, type of disease, the kind of crystal, etc. but generally speaking about 1 mg to about 4000 mg/man, or even more, can be administered daily to the patient. For oral administration, as an example, the crystal of Compound (I) or a salt thereof according to this invention can be administered in a unit dose of about 10 mg, 50 mg, 100 mg, 250 mg, 500 mg or 1000 mg, in such dosage forms as tablets, granules, powders or capsules for the treatment of infectious diseases associated with pathogenic bacteria.

As evidence of the usefulness of the crystal of Compound (I) or a salt thereof, its stability, antibacterial activity, and urinary excretion data are shown below.

### (A) Stability test

### (1) Test samples

The water-containing crystals and isopropyl alcohol and water-containing crystals of Compound (I) or a salt thereof obtained in Examples 5, 7 and 8(1) were tested.

### Test method

The one-month stability of each test sample in an airtight container at 40°C was evaluated.

The potency of each test sample was determined by liquid chromatography and the percentage residue of Compound (I) or a salt thereof was calculated.

**[Table 1]**

| | Crystal of Example 5 | Crystal of Example 7 | Crystal of Example 8(1) |
|---|---|---|---|
| Residue (%) | 98.6 | 99.2 | 98.5 |

The above results indicate that the crystals of Compound (I) or a salt thereof obtained in accordance with this invention are sufficiently stable

### (2) Test samples

Four lots of the isopropyl alcohol and water-containing crystal of compound (I) as obtained in Example 8 (2) were used.

### Test method

The 9-day stability of each test sample in an airtight container at 70°C was determined. The potency of each test sample was determined by liquid chromatography and the percentage residue was calculated. The results are shown in Table 2.

**[Table 2]**

| Lot No. | Residue (%) |
|---|---|
| 35414OP | 98.7 |
| 37914XP | 98.0 |
| 35434OP | 98.0 |
| 35534OP | 99.2 |

### (B) Minimal inhibitory concentration

Compound (I) or a salt thereof exhibits high antibacterial activity against a broad spectrum of pathogenic bacteria ranging from gram-positive bacteria to gram-negative bacteria, thus being of value as an antibacterial agent.

For proof of the usefulness of Compound (I) or a salt thereof, its MIC (minimal inhibitory concentration) data are presented below.

### Assay method:

The in vitro antibacterial activity was determined by the following doubling dilution assay using agar plates.

Each test strain of microorganism was cultured in Tryptocase soy broth overnight and a loopful (viable count 10% cells/ml) of the culture was used to inoculate heart infusion agar (HI-agar) plates containing a concentration series of Compound (I). The inoculated plates were incubated at 37°C for 20 hours and the minimal inhibitory concentrations (MIC) were determined and expressed in µg/ml. Results

**[Table 3]**

| MIC (µg/ml) | |
|---|---|
| Test strain | Compound (I) |
| S. aureus | 0.23 |
| E. coli | 0.033 |
| H. influenzae | 0.129 |

### (C) Urinary excretion

### Test method

Male SD rats (6-7 weeks old) from JCL were used. The test compound was suspended in 0.5% methylcellulose solution. The rats were fasted overnight before administration of 20 mg/kg. The urine was collected in the time slots of 0-6 and 6-24 hours after oral administration and the urinary recovery was determined by the disk plate diffusion method using Bacillus subtilis ATCC 6633 as the test organism and sodium citrate agar (0.8% sodium citrate, 0.5% polypeptone, 0.3% beef extract, and 1.0% agar) as the test medium.

### Result

**[Table 4]**

| | Recovery in 24-hrs urine (%) |
|---|---|
| Compound (I) | 50.0 |

The abbreviations used in the production examples and working examples have the following meanings.
- THF:: tetrahydrofuran
- IPE:: diisopropyl ether
- DMF:: N,N-dimethylformamide
- HP-20:: the trademark of a porous resin
- FAB-MASS:: fast atom bombardment mass spectrometry

### Brief Description of the Drawings

Fig. 1 is a powder X-ray diffraction pattern of the crystals obtained in Example 4.

Fig. 2 is a powder X-ray diffraction pattern of the crystals obtained in Example 5.

Fig. 3 is a powder X-ray diffraction pattern of the crystals obtained in Example 6.

Fig. 4 is a powder X-ray diffraction pattern of the crystals obtained in Example 7.

Fig. 5 is a powder X-ray diffraction pattern of the crystals obtained in Example 8(1).

Fig. 6 is a powder X-ray diffraction pattern of the crystals obtained in Example 9.

The following Preperations and Examples are shown to describe this invention in further detail.

### Preperation 1

To a solution of potassium t-butoxide (383 g) in DMF (3.5 L) was added 4-(methoxycarbonyl)pyrazole (360 g) portionwise under ice-cooling. After stirring for 20 minutes, trityl chloride (794 g) was added gradually while the mixture was maintained at a temperature not exceeding 20°C. This reaction mixture was poured into water-ethyl acetate (water 14 L / ethyl acetate 28 L). The organic layer was seperated, washed serially with 2 portions of water (7 L each) and 20% sodium chloride solution (7 L), and dried over magnesium sulfate. The solvent was then distilled off to provide 4-methoxycarbonyl-1-(trityl)pyrazole (1021 g).

### Preperation 2

A solution of 4-methoxycarbonyl-1-(trityl)pyrazole (600 g) in THF (3.5 L) was added portionwise to a suspension of lithium aluminum hydride (61.8 g) in THF (2.0 L). After stirring for 1 hour, water (62 ml) was added. The mixture was further diluted with 15% sodium hydroxide solution (62 ml) and water (186 ml) and filtered. The residue was washed with chloroform-methanol (10/1; 2 L). The filtrate was concentrated under reduced pressure. The residual powder was dissolved in methylene chloride (6 L) and the solution was washed with 10% sodium chloride solution (1 L). The organic layer was taken and dried over magnesium sulfate (400 g) and the solvent was distilled off. The residual powder was collected, triturated with 2 portions of IPE (2 L each) and air-dried overnight to provide 4-hydroxymethyl-1-(trityl)pyrazole (412 g).

### Preparation 3

Under nitrogen atmosphere, 10.0 ml of triethylamine (72.6 m mol) and 4.1 ml of methanesulfonyl chloride (53 m mol) was added successively to a solution of 6.27 g of 5-hydroxymethyl-4-methyl-1,2,3-thiadiazole (48.16 m mol) in dichloromethane (50 ml) at -30°C. After stirring for 30 minutes, the mixture was poured into water-dichloromethane while the pH was kept between 8.5-9.0. The organic layer was separated and washed with saturated sodium hydrogencarbonate, dil-hydrochloric acid and brine, dried over magnesium sulfate. After filtration, the filtrate was concentrated in vacuo to afford 5-methanesulfonyloxymethyl-4-methyl-1,2,3-thiadiazol (9.8 g).
NMR (CDCl₃, δ) : 2.76 (3H, s), 3.06 (3H, s), 5.51 (2H, s)

### Preparation 4

The following compounds were obtained according to a similar manner to that of Preparation 3.
1-Trityl-4-(methanesulfonyloxymethyl)pyrazole
NMR (CDCl₃, δ) : 2.92 (3H, s), 4.46 (2H, s), 7.05-7.50 (5H, m), 7.42 (1H, s), 7.65 (1H, s)

### Preparation 5

Under nitrogen atmosphere, 6.64 ml (56.5 m mol) of thiobenzoic acid was added to a stirred solution of potassium tert-butoxide (6.07 g, 54.1 m mol) in DMF (80 ml) at 0°C. After stirring for 10 minutes, 5-methanesulfonyloxymethyl-4-methyl-1,2,3-thiadiazole (9.8 g, 47.0 m mol) in DMF (30 ml) was added to the mixture slowly at the sane temperature. The whole mixture was stirred at 80°C for 2 hours, poured into a mixture of diluted aqueous sodium hydrogencarbonate and ethyl acetate. The organic layer was separated and washed with brine, and dried over magnesium sulfate. After evaporation of the solvent, the residue was treated on silica gel (eluent: n-hexane/ethyl acetate = 9/1 - 8/2) to afford 5-benzoylthiomethyl-4-methyl-1,2,3-thiadiazole (7.51 g).
NMR (CDCl₃, δ) : 2.73 (3H, s), 4.47 (2H, s), 7.42-7.65 (3H, m), 7.90-7.97 (2H, m)

### Preparation 6

The following compounds were obtained according to a similar manner to that of Preparation 5.
4-Benzoylthiomethyl-1-(trityl)pyrazole
NMR (CDCl₃, δ) : 4.15 (2H, s), 7.05-7.65 (20H, m), 7.90-8.00 (2H, m)

### Preperation 7

Under nitrogen atmosphere, 1.35 ml of sodium methoxide (6.5 m mol) was added slowly to a solution of 1.50 g of 1-methyl-4-benzoylthiomethylpyrazole in THF (6 ml) and DMF (18 ml) at 0°C. Stirring was continued for 1 hour. The mixture was cooled to -65°C with dry ice/ethanol bath, and added to a solution of 4.36 g of diphenylmethyl 7β-[2-(2-aminothiazol-4-yl)-2-(Z)-(trityloxyimino)acetamido]-3-methanesulfonyloxy-3-cephem-4-carboxylate in a mixture of THF (15 ml) and DMF (25 ml) at the same temperature. After stirring for 1 hour, the reaction was quenched with 10% hydrochloric acid, and the mixture was poured into water - ethyl acetate. The organic layer was separated, washed with brine, dried over magnesium sulfate. After filtration, the filtrate was concentrated in vacuo, the residue was purified on silica gel (eluent: dichloromethane-acetone) to afford diphenylmethyl 7β-[2-(2-aminothiazol-4-yl)-2-(Z)-(trityloxyimino)acetamido]-3-[(1-methylpyrazol-4-yl)methylthio]-3-cephem-4-carboxylate (2.15 g).
NMR (DMSO-d₆, δ) : 3.71 (3H, s), 3.87 (2H, s), 4.05 (2H, d, J=4.3Hz), 5.31 (1H, d, J=4.62Hz), 5.93 (1H, dd, J=8.52 and 4.54Hz), 6.70 (1H, s), 6.83 (1H, d, J=2.36Hz), 6.86 (1H, s), 7.19-7.59 (26H, m), 9.88 (1H, d, J=8.52Hz)

### Preperation 8

The following compounds were obtained according to a similar manner to that of Preperation 7.
Diphenylmethyl 7β-[2-(2-aminothiazol-4-yl)-2-(Z)-(trityloxyimino)acetamido]-3-[(1-tritylpyrazol-4-yl)methylthio]-3-cephem-4-carboxylate
NMR (DMSO-d₆, δ) : 3.81 (2H, s), 4.07 (2H, s), 5.25 (1H, d, J=4.6Hz), 5.91 (1H, dd, J=8.4 and 4.6Hz), 6.71 (1H, s), 6.86 (1H, s), 6.99-7.03 (6H, m), 7.20-7.57 (36H, m), 9.87 (1H, d, J=8.6Hz)

### Preperation 9

Under nitrogen atomosphere, a solution of aluminium chloride (2.65 g) in anisole (5.7ml) was added dropwise to a solution of diphenylmethyl 7β-[2-(2-aminothiazol-4-yl)-2-(Z)-(trityloxyimino)acetamido]-3-[(1-tritylpyrazol-4-yl)methylthio]-3-cephem-4-carboxylate (3.32 g) in a mixture of anisole (5.7 ml) and nitromethane (22.5 ml) at -24°C. After stirring for 1 hour at the same temperature, the reaction was quenched with 1N hydrochloric acid (22.5 ml). The mixture was poured into a mixture of water and ethyl, acetate. The aqueous layer was separated and the organic layer was reextracted with water. The combined aqueous layer was concentrated in vacuo, chromatographed on a HP-20 column (eluent: water - methanol). After the concentration, the resulting precipitate was collected by filtration to afford 7β-[2-[2-aminothiazol-4-yl)-2-(Z)-(hydroxyimino)acetamido]-3-[(pyrazol-4-yl)methylthio]-3-cephem-4-carboxylic acid (133.1 mg)
IR (KBr) : 3203, 1762, 1660, 1600 cm⁻¹
NMR (DMSO-d₆, δ) : 3.76 (2H, s), 4.02 (2H, d, J=2Hz), 5.14 (1H, d, J=4.6Hz), 5.69 (1H, dd, J=8.2 and 4.6Hz), 6.68 (1H, s), 7.13 (1H, s), 7.55 (1H, s), 9.46 (1H, d, J=8.3Hz), 11.30 (1H, s)

### Preperation 10

After triethylamine (194ml) was added to a solution of 4-hydroxymethyl-1-(trityl)pyrazole (400g) in dichloromethane (5.2 L) under ice-cooling, methanesulfonylchloride was added potionwise to the mixture. After stirring for 40 minutes at room temperature, to the reaction mixture was added water (2 L). The organic layer was separated, added water (2 L), and adjusted to pH 3 - 4 with 6N hydrochloric acid. The organic layer was separated, was washed 5% sodium chloride aqueous solution (2 L), dried over magnesium sulfate and the solvent was distilled away. The resulting residue was dissolved in dichloromethane (500 ml) and treated on silica gel (eluent: n-hexane/ethyl acetate = 9/1 - 7/3) to afford 4-chloromethyl-1-tritylpyrazole(245.1g).

### Preparation 11

Suspension A; 4-Chloromethyl-1-tritylpyrazole (198.3 g) was suspended in acetone (3.0 ℓ) and it was warmed at 50°C. After it was dissolved, sodium iodide (165.6 g) was added to the solution at the room temperature. The solution was stirred at the same temperature for an hour, and then it was poured into a mixture of ethyl acetate (3.0 ℓ) and water (3.0 ℓ). The organic layer was separated, dried over magnesium sulfate and evaporated. The residue was suspended in DMF (400 ml) to give suspension A.

Suspension B; On the other hand, under N₂ atmosphere 70% sodium hydrosulfide (36.1 g) was suspended in DMF (0.6 ℓ) at the room temperature, N,N-diisopropylethylamine (107 ml) was added to the suspension to give suspension B.

The solution of diphenylmethyl 7β-formamido-3-methanesulfonyloxy-3-cephem-4-carboxylate (200 g) in DMF (1.6 ℓ) was cooled to -2°C, suspension B was dropped into the solution below 0°C for 40 minutes. After stirring at the same temperature for one hour, the suspension A was dropped into the solution below 0°C, and stirred at the same temperature for 30 minutes. The reaction mixture was poured into a mixture of ethyl acetate (7 ℓ) and water (7 ℓ), aqueous layer was adjusted to pH 6.5 with 3N-hydrochloric acid. Organic layer was separated, washed with water (4 ℓ). The organic layer was left at 5°C for 14 hours. The resulting precipitate was collected by filtration, washed with ethyl acetate (1.5 ℓ) to give diphenylmethyl 7β-formamido-3-[(1-tritylpyrazol-4-yl)methylthio]-3-cephem-4-carboxylate (242 g) as powder.
IR (KBr) : 1772, 1732, 1693, 1660, 1375, cm⁻¹
NMR (DMSO-d₆, δ) : 3.81 (2H, s), 4.05 (2H, s), 5.12 (1H, d, J=4.6Hz), 5.74 (1H, dd, J=4.6Hz, J=8.7Hz), 6.84 (1H, s), 7.00-7.56 (27H, m), 8.19 (1H, s), 9.12 (1H, d, J=8.7Hz)
FAB-Mass : 748 (M⁺)

### Preparation 12

The following compound was obtained according to a similar manner to that of Preparation 11.
Diphenylmethyl 7β-phenylacetamido-3-[(1-tritylpyrazol-4-yl)methylthio]-3-cephem-4-carboxylate
IR (KBr) : 1781, 1685, 1533, 1496 cm⁻¹
NMR (DMSO-d₆, δ) : 3.51 and 3.61 (2H, ABq, J=18Hz), 3.81 (2H, br s), 4.01 (2H, br s), 5.07 (1H, d, J=5Hz), 5.65 (1H, dd, J=5Hz, 7Hz), 6.84 (1H, s), 7.00-7.60 (32H, m), 9.16 (1H, d, J=7Hz)

### Preparation 13

Under nitrogen atmosphere, a solution of diphenylmethyl 7β-formamido-3-methanesulfonyloxy-3-cephem-4-carboxylate (2.44 g) in DMF (15 ml) was added to the mixture of 440 mg of sodium hydrosulfide and 1.3 ml of disopropylethylamine in DMF (10 ml) under cooling with dry ice-tetrachloromethane. Stirring was continued for 30 minutes, 918 mg of 4-chloromethylpyrazole hydrochloride and 1.04 ml of diisopropylethylamine was added successively to the solution. The whole mixture was stirred for 1 hour, and then poured into a mixture of water and ethyl acetate. Organic layer was separated, and washed-with diluted hydrochloric acid and brine, successively, and dried over magnesium sulfate. After evaporation of the solvent, the residue was purified on silica gel (eluent : a mixture of dichloromethane and acetone) to afford diphenylmethyl 7β-formamido-3-[(pyrazol-4-yl)-methylthio]-3-cephem-4-carboxylate (2.96 g).
IR (KBr) : 3303.5, 1791.5, 1760.7, 1672.0, 1535.1 cm⁻¹
NMR (DMSO-d₆, δ) : 3.88 (2H, s), 4.05, 4.09 (2H, ABq, J=13.2Hz), 5.19 (1H, d, J=4.6Hz), 5.74 (1H, dd, J=9.0Hz, 4.6Hz), 6.84 (1H, s), 7.20-7.80 (12H, m), 8.18 (1H, s), 9.12 (1H, d, J=9.4Hz), 12.78 (1H, s)

### Preparation 14

The following compound was obtained according to a similar manner to that of Preparation 13.
Diphenylmethyl 7β-phenylacetamido-3-[(pyrazol-4-yl)methylthio]-3-cephem-4-carboxylate
IR (KBr) : 1772, 1716, 1648, 1558, 1496 cm⁻¹
NMR (DMSO-d₆, δ) ; 3.51 and 3.60 (2H, ABq, J=18Hz), 3.88 (2H, br s), 4.03 and 4.10 (2H, ABq, J=18Hz), 5.15 (1H, d, J=5Hz), 5.65 (1H, dd, J=5Hz, 7Hz), 6.84 (1H, s), 7.10-7.65 (17H, m), 9.18 (1H, d, J=7Hz)

### Preparation 15

Diphenylmethyl 7β-formamido-3-[(1-tritylpyrazol-4-yl)methylthio]-3-cephem-4-carboxylate (121.7 g) was suspended in methanol (1.46 ℓ), concentrated hydrochloric acid (94.8 ml) was added thereto below 25°C. The reaction mixture was stirred at the room temperature for 3 hours, and then concentrated hydrochloric acid (4.0 ml) was added. After the reaction mixture was stirred at the same temperature for one hour, insoluble precipitate was filtered off below 10°C. The filtrate was poured into a mixture of ethyl acetate (4.5 ℓ) and water (4 ℓ). The aqueous layer was adjusted at pH 4.0 with 30% aqueous sodium hydroxide solution and then was adjusted at pH 6.9 with 2N-potassium hydroxide solution. The organic layer was separated, washed with brine (4 ℓ), dried over magnesium sulfate, and evaporated until the volume amounted to 700 ml. IPE (100 ml) was added to the suspension gradually below 10°C, and was left below 10°C for 12 hours. The precipitate was filtered and dried under reduced pressure to give diphenylmethyl 7β-amino-3-[(pyrazol-4-yl)methylthio]-3-cephem-4-carboxylate (62.4 g) as powder.
IR (KBr) : 1743, 1697, 1369, 1213 cm⁻¹
NMR (DMSO-d₆, δ) : 2.34 (2H, s), 3.84 (2H, s), 4.01 (2H, s), 4.79 (1H, s), 5.02 (1H, d, J=4.9Hz), 6.82 (1H, s), 7.24-7.59 (12H, m), 12.76 (1H, s)
FAB-Mass : 479 (M⁺+1)

| Elemental Analysis Calcd. for C₂₄H₂₂N₄O₃S₂ : | | | |
|---|---|---|---|
| | C 60.23, | H 4.63, | N 11.71 |
| Found : | C 60.33, | H 4.88, | N 11.63 |

### Preparation 16

Pyridine (1.3 ml) was added to a suspension of phosphorus pentachloride (3.37 g) in dichloromethane (47.6 ml) at -10°C, and the mixture was stirred at between -15 to -5°C for 30 minutes. Diphenylmethyl 7β-phenylacetamido-3-[(1-tritylpyrazol-4-yl)methylthio]-3-cephem-4-carboxylate (6.8 g) was added to the above mixture at -10°C and the reaction mixture was stirred under ice-cooling for 1 hour. Then, methanol (5.2 ml) was added to the reaction mixture at -20°C and the resulting solution was stirred under ice-cooling for 1 hour. Water (40 ml) was added to the above mixture under ice-cooling, and stirred for 30 minutes at the same temperature. The aqueous layer was separated and the dichloromethane layer was reextracted with 1 mol hydrochloric acid (30 ml). The aqueous layer and 1 mol hydrochloric acid layer were combined. Ethyl acetate (50 ml) was added to the aqueous layer and then the mixture was adjusted to pH 3.5 with 30% aqueous sodium hydroxide under stirring. The organic layer was separated, washed with brine and dried over magnesium sulfate. The solvent was distilled off under reduced pressure and the residue was pulverized with IPE (50 ml), collected by filtration, washed with IPE (20 ml) and dried over phosphorus pentoxide to give powder of diphenylmethyl 7β-amino-3-[(pyrazol-4-yl)methylthio]-3-cephem-4-carboxylate (2.1 g).

The physical data showed that the object compound is the same with the object compound of the Preparation 15.

### Preparation 17

Diphenylmethyl 7β-amino-3-[(pyrazol-4-yl)methylthio]-3-cephem-4-carboxylate was obtained from diphenylmethyl 7β-formamido-3-[(pyrazol-4-yl)-methylthio]-3-cephem-4- carboxylate according to a similar manner to that of Preparation 16.

The physical data showed that the object compound is the same with the object compound of the Preparation 15.

### Preparation 18

Diphenylmethyl 7β-formamido-3-[(pyrazol-4-yl)methylthio]-3-cephem-4-carboxylate (2.96 g) was dissolved in methanol (30 ml) and concentrated hydrochloric acid (2.2ml) was added thereto at the room temperature. Stirring was continued for 3 hours, then solvent was evaporated. The residue was diluted with a mixture of water and the ethyl acetate. The aqueous layer was adjusted to pH 6.5 with 30% aqueous potassium carbonate. The organic layer was separated, washed with water and brine succesively, and dried over magnesium sulfate. Solvent was evaporated to afford diphenylmethyl-7β-amino-3-[(pyrazol-4-yl)methylthio]-3-cephem-4-carboxylate (1.50 g).

The physical data showed that the object compound is the same with the object compound of the Preparation 15.

### Preperation 19

Under nitrogen atmosphere, to a suspension of diphenylmethyl 7β-amino-3-[(pyrazol-4-yl)methylthio]-3-cephem-4-carboxylate (30.2 g) in THF (800 ml) was added herein 1,3-bis(trimethylsilyl)urea (25.8 g) at room temperature. The reaction mixture was warmed at 35°C and dissolved, and then it was cooled below 0°C. A suspension of 2-(2-aminothiazol-4-yl)-2-(Z)-(acetoxyimino)acetylchloride monohydrochloride salt (17.93 g) in acetonitrile (200 ml) was dropped into the above reaction mixture below 0°C. After stirring at the same temperature for 10 minutes, it was poured into a mixture of ethyl acetate (1.2 ℓ) and ice-water (1.5 ℓ). The aqueous layer was adjusted at pH 6.5 with saturated sodium bicarbonate solution. The organic layer was separated, washed with brine (1.0 ℓ), and dried over magnesium sulfate, and then evaporated until the volume amounted to 500 ml. The solution was poured into IPE (1.5 ℓ). The resulting precipitate was filtered, dried under reduced pressure to give diphenylmethyl 7β-[2-(2-aminothiazol-4-yl)-2-(Z)-(acetoxyimino)acetamido]-3-[(pyrazol-4-yl)methylthio]-3-cephem-4-carboxylate (41.3 g) as powder.
IR (KBr) : 1772, 1684, 1616, 1533, 1375, 1219 cm⁻¹
NMR (DMSO-d₆, δ) : 2.17 (3H, s), 3.83-4.07 (4H, m), 5.26 (1H, d, J=4.6Hz), 5.82 (1H, dd, J=4.6Hz, 8.2Hz), 6.83 (1H, s), 7.13 (1H, s), 7.23-7.52 (14H, m), 9.90 (1H, d, J=8.2Hz), 12.75 (1H, s)
FAB-Mass : 690 (M⁺+1)

### Preparation 20

Diphenylmethyl 7β-[2-(2-aminothiazol-4-yl)-2-(Z)-(acetoxyimino)acetamido]-3-[(pyrazol-4-yl)methylthio]-3-cephem-4-carboxylate (41.3 g) was suspended in methanol (420 ml) at room temperature, concentrated hydrochloric acid (24.9 ml) was added below 15°C thereto. After the reaction mixture was stirred at room temperature for 30 minutes, concentrated hydrochloric acid (6.7 ml) was added thereto at the sane temperature. After stirring at room temperature for 2 hours, poured into a mixture of ethyl acetate (1.2 ℓ) and pH 6.86 buffer (1.5 ℓ). The pH was adjusted to pH 5.0 with 30% aqueous sodium hydroxide, and then was adjusted to pH 6.0 with 2N-potassium hydroxide. The organic layer was separated, and THF (0.5 ℓ) was added thereto. The organic layer was washed with brine (1.0 ℓ), dried over magnesium sulfate, and evaporated until the volume amounted to 500 ml. A mixture of IPE (500 ml) and ethyl acetate (700 ml) was added thereto. Resulting precipitate was filtered, dried under reduced pressure to afford diphenylmethyl 7β-[2-(2-aminothiazol-4-yl)-2-(Z)-(hydroxyimino)acetamido]-3- [(pyrazol-4-yl)methylthio]-3-cephem-4-carboxylate (33.6 g) as powder.
IR (KBr) : 1772, 1684, 1616, 1533 cm⁻¹
NMR (DMSO-d₆, δ) : 3.78-4.07 (4H, m), 5.22 (1H, d, J=4.6Hz), 5.79 (1H, dd, J=4.6Hz, 8.4Hz), 6.84 (1H, s), 7.14 (1H, s), 7.24-7.53 (14H, m), 9.50 (1H, d, J=8.4Hz), 11.32 (1H, s)
FAB-Mass : 648 (M⁺+1)

### Preparation 21

Under nitrogen atmosphere, 1.7 ml of thionyl chloride was added to a suspension of 1.0 g of 4-hydroxymethylpyrazole in chloroform (25 ml) at room temperature. Stirring was continued for 30 minutes and the solvent was evaporated. The residue was washed with ether and dried under vacuo to afford 4-chloromethylpyrazole hydrochloride (1.29 g).
NMR (DMSO-d₆, δ) : 4.74 (2H, s), 7.96 (2H, s)

### Example 1

Under nitrogen atmosphere, diphenylmethyl 7β-[2-(2-aminothiazol-4-yl)-2-(Z)-(hydroxyimino)acetamido]-3-[(pyrazol-4-yl)methylthio]-3-cephem-4-carboxylate (33.5 g) was suspended in dichloromethane (100 ml) and anisole (35 ml). Trifluoroacetic acid (80 ml) was added dropwise below 5°C for 40 minutes. After stirring below 5°C for 25 minutes, the reaction mixture was poured into IPE (1.8 ℓ). Resulting precipitate was collected by filtration and dried under reduced pressure. The powder was poured into pH 6.86 buffer (550 ml). The suspension was adjusted to pH 6.9 with 2N-potassium hydroxide, then was stirred at 15°C until insoluble material disappeared. The solution was subjected to column chromatography on HP-20 (700 ml). The column was washed with water (1.4 ℓ) and the object compound was eluted with 25% aqueous 2-propanol. The active fractions were collected, and adjusted to pH 3.5 with 3N-hydrochloric acid. After stirring at 30°C for 2 hours, resulting precipitate was filtered and washed with water (50 ml) two times. The precipitate was suspended in water (150 ml), and adjusted to pH 2.0 with 1N-hydrochloric acid. After stirring at room temperature for one hour, the precipitate was collected and washed with water (20 ml). The precipitate was suspended in water (153 ml) again, and then adjusted to pH 2.0 with 1N hydrochloric acid. After stirring at room temperature for one hour, it was adjusted to pH 2.8 with 2N potassium hydroxide. After stirring at the same temperature for 30 minutes, the precipitate was collected, washed with water (20 ml), and dried under reduced pressure to afford 3.75 hydrates or 7β-[2-(2-aminothiazol-4-yl)-2-(Z)-(hydroxyimino)acetamido]-3-[(pyrazol-4-yl)methylthio]-3-cephem-4-carboxylic acid as crystal (9.7 g).
IR (KBr) : 1763, 1647, 1603, 1541 cm⁻¹
NMR (DMSO-d₆, δ) : 3.69, 3.74 (2H, ABq, J=14.2Hz), 3.99, 4.06 (2H, ABq, J=13.4Hz), 5.15 (1H, d, J=4.6Hz), 5.69 (1H, dd, J=4.6Hz, 8.2Hz), 6.71 (1H, s), 7.30 (2H, s), 7.56 (2H, s), 9.48 (1H, d, J=8.2Hz), 11.41 (1H, s)
FAB-Mass : 481 (M⁺)

| Elemental Analysis Calcd. for C₁₆H_{22.5}N₇O_{8.75}S₃ : | | | | |
|---|---|---|---|---|
| | C 35.00, | H 4.13, | N 17.86, | S 17.52 |
| Found : | C 34.71, | H 3.84, | N 17.79, | S 17.30 |

| Philips MPD 1880 X-Ray Powder Diffraction System | |
|---|---|
| 2θ | intensity |
| 7.5 | 620 |
| 8.5 | 150 |
| 11.4 | 370 |
| 17.1 | 160 |
| 18.7 | 150 |
| 19.5 | 410 |
| 19.7 | 300 |
| 20.8 | 250 |
| 21.4 | 390 |
| 23.1 | 180 |
| 24.6 | 520 |
| 25.8 | 290 |
| 29.0 | 230 |
| X-ray : Monochlomated CuKα radiation Voltage : 40 KV / Current : 30mA | |

### Example 2

To a suspension of 7β-[2-(2-aminothiazol-4-yl)-2-(Z)-(hydroxyimino)acetamido]-3-[(pyrazol-4-yl)methylthio]-3-cephem-4-carboxylic acid (337 g) in water (6.7 ℓ) was added sodium hydrogencarbonate (69.7 g) at room temperature. After stirring at 30°C till the mixture turned clear solution, a mixture of 1N-hydrochloric acid (100 ml) and 2-propanol (3.0 ℓ) was added to thereto at room temperature. The PH of the solution was adjusted to pH 4.0 with 1N-hydrochloric acid (350 ml), the suspension was stirred for one hour at 30°C. The pH of the solution was adjusted to pH 3.3 with 1N-hydrochloric acid (185 ml), and then cooled with ice-bath. After stirring for 2 hours below 10°C, the resulting precipitate was filtered, washed with water (3 ℓ) and 2-propanol (2 ℓ) successively. The precipitate was dried with vacua at 40°C for 5 hours and then at room temperature for 14 hours to afford 329g of 7β-[2-(2-aminothiazol-4-yl)-2-(Z)-(hydroxyimino)acetamido]-3-[(pyrazol-4-yl)methylthio]-3- cephem-4-carboxylic acid containing 1/3 isopropanol as crystal.
IR (KBr) : 3263, 3111, 2925, 2854, 1774, 1645, 1601, 1551, 1508, 1346 cm⁻¹
NMR (DMSO-d₆, δ) : 1.02 (1H, s), 1.05 (1H, s), 3.70, 3.76 (2H, ABq, J=14.2Hz), 3.98, 4.06 (2H, ABq, J=13.4Hz), 4.30-4.40 (0.33H, m), 5.14 (1H, d, J=4.6Hz), 5.69 (1H, dd, J=4.6Hz, J=8.2Hz), 6.69 (1H, s), 7.13 (2H, s), 7.55 (2H, s), 9.45 (1H, d, J=8.2Hz), 11.30 (1H, s)

| Philips MPD 1880 X-Ray Powder Diffraction System | |
|---|---|
| 2θ | intensity |
| 7.5 | 570 |
| 8.5 | 150 |
| 11.2 | 270 |
| 17.1 | 200 |
| 18.6 | 160 |
| 19.4 | 390 |
| 19.6 | 250 |
| 20.6 | 190 |
| 21.2 | 380 |
| 22.9 | 250 |
| 24.4 | 400 |
| 25.5 | 300 |
| 25.9 | 220 |
| 28.9 | 230 |
| X-ray : Monochlomated CuKα radiation Voltage : 40 KV / Current : 30mA | |

This crystal was stable in the stability test.

### Example 3

7β-[2-(2-Aminothiazol-4-yl)-2-(Z)-(hydroxyimino)acetamido]-3-[(pyrazol-4-yl)methylthio]-3-cephem-4-carboxylic acid (4.65 g) was suspended in water (27.9 ml) at room temperature, and 1N hydrochloric acid (19.3 ml) was added thereto. After stirring at 40°C for 5 minutes, and then 1N hydrochloric acid (1.60 ml), water (4.0 ml) and ethanol (9.0 ml) was added therein at the same temperature. After stirring at 40°C for three minutes, the mixture was further stirred at room temperature for three hours. Resulting crystal was collected by filtration, washed with water (10 ml) two times, and dried under reduced pressure to afford 7β-[2-(2-aminothiazol-4-yl)-2-(Z)-(hydroxyimino)acetamido]-3-[(pyrazol-4-yl)methylthio]-3-cephem-4-carboxylic acid 1/2 hydrochloride 3 hydrates(2.4 g) as crystals.
IR (KBr) : 1770, 1734, 1670, 1541 cm⁻¹
NMR (DMSO-d₆, δ) : 3.71, 3.82 (2H, ABq, J=17.0Hz), 3.99, 4.07 (2H, ABq, J=13.4Hz), 5.16 (1H, d, J=4.6Hz), 5.68 (1H, dd, J=4.6Hz, 8.0Hz), 6.80 (1H, s), 7.56 (2H, s), 9.59 (1H, d, J=8.0Hz), 11.82 (1H, s)

| Elemental Analysis Calcd. for C₁₆H_{21.5}Cl_{0.5}N₇O₈S₃ : | | | | | |
|---|---|---|---|---|---|
| | C 34.70, | H 3.91, | N 17.70, | Cl 3.20, | S 17.37 |
| Found : | C 34.85, | H 3.70, | N 17.97, | Cl 3.09, | S 17.26 |

### Example 4

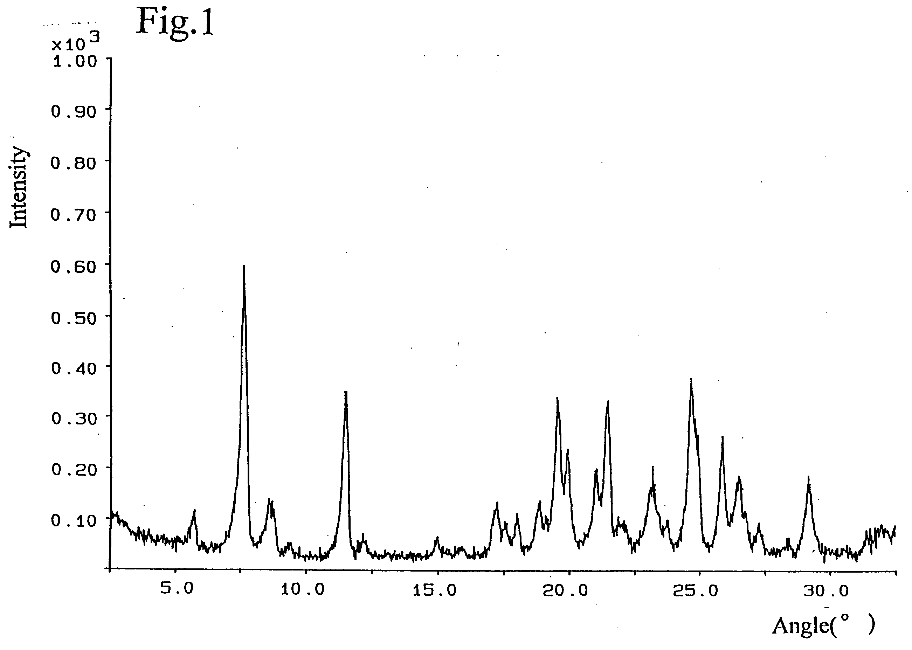

The noncrystalline form of 7β-[2-(2-aminothiazol-4-yl)-2-(z)-(hydroxyimino)acetamido]-3-[(pyrazol-4-yl)methylthio]-3-cephem-4-carboxylic acid (20.0 g) obtained in Preperation 9 was suspended in water (340 ml) and the suspension was cooled to 5-15°C. Then, sodium hydrogencarbonate (3.6 g) was added and the mixture was stirred at the same temperature for 1 hour to prepare a homogeneous solution, which was then filtered. To the filtrate was added ethanol (200 ml) and the mixture was warmed to 25-30°C and adjusted to pH 3.8-4.3 with 1N-hydrochloric acid (25 ml). While the pH was maintained at 3.8-4.3 with 1N-HCl, the solution was stirred for 10 hours. The resulting precipitate was collected by filtration and washed with water (200 ml) and ethyl alcohol (100 ml). The washed precipitate was dried under reduced pressure to provide crystals of 7β-[2-(2-aminothiazol-4-yl)-2-(Z)-(hydroxyimino)acetamido]-3-[(pyrazol-4-yl)methylthio]-3-cephem-4-carboxylic acid containing water (2.1 molecules) and ethyl alcohol (0.37 molecule). The powder X-ray diffraction pattern of the crystals thus obtained is shown in Fig. 1.

### Example 5

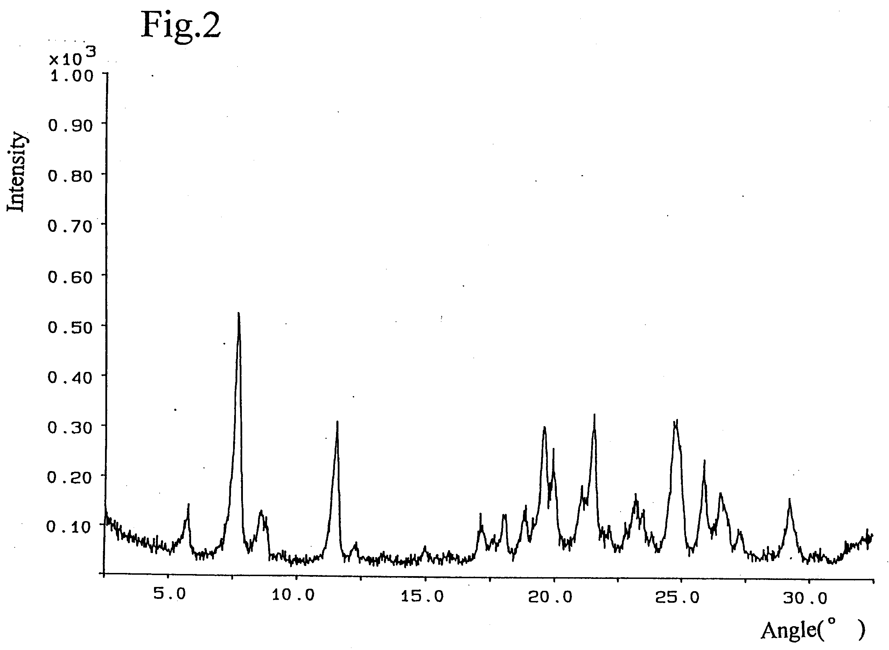

Using streams of 35-50% moisture-laden air, the crystals obtained in Example 4 (all volume) were dried at 35°C for 2 days to remove the solvent, followed by drying under reduced pressure to provide crystals (16.5 g) of 7β-[2-(2-aminothiazol-4-yl)-2-(Z)-(hydroxyimino)acetamido]-3-[(pyrazol-4-yl)methylthio]-3-cephem-4-carboxylic acid containing 2.5 molecules of water. The powder X-ray diffraction pattern of the crystals thus obtained is shown in Fig. 2.

### Example 6

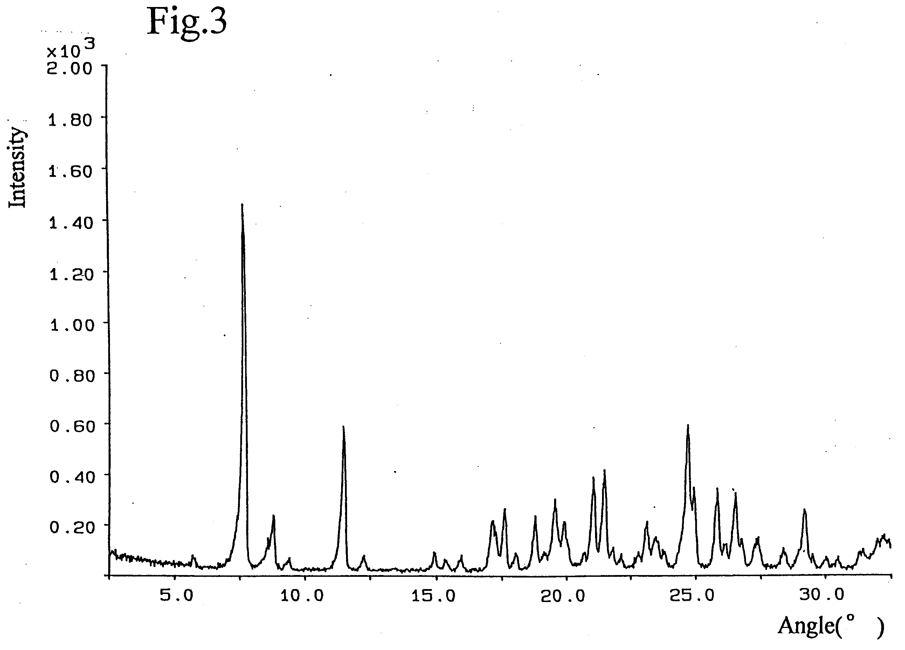

The 7β-[2-(2-aminothiazol-4-yl)-2-(Z)-(-hydroxyimino)acetamido]-3-[(pyrazol-4-yl)methylthio]-3-cephem-4-carboxylic acid (20.0 g) obtained in Preperation 9 was suspended in water (340 ml) and the suspension was cooled to 5-15°C. To this cooled suspension was added sodium hydrogencarbonate (3.6 g) and the mixture was stirred at the same temperature for 1 hour to prepare a homogeneous solution, which was then filtered. To the filtrate was added acetone (200 ml) and the mixture was warmed to 25-30°C and adjusted to pH 4.5 with 1N-hydrochloric acid (14 ml). When this solution was seeded with the crystals obtained in Preparation 9, it undergoes crystallization gradually. This solution was further stirred for 10 hours while its pH was controlled at 3.8-4.3. The resulting precipitate is collected by filtration, washed with water (200 ml) and acetone (100 ml), and dried under reduced pressure to provide crystals of 7β-[2-(2-aminothiazol-4-yl)-2-(Z)-(-hydroxyimino)acetamido]-3-[(pyrazol-4-yl)methylthio]-3-cephem-4-carboxylic acid containing water (2.2 molecules) and acetone (0.37 molecule). The powder X-ray diffraction pattern of the crystals thus obtained was shown in Fig. 3.

### Example 7

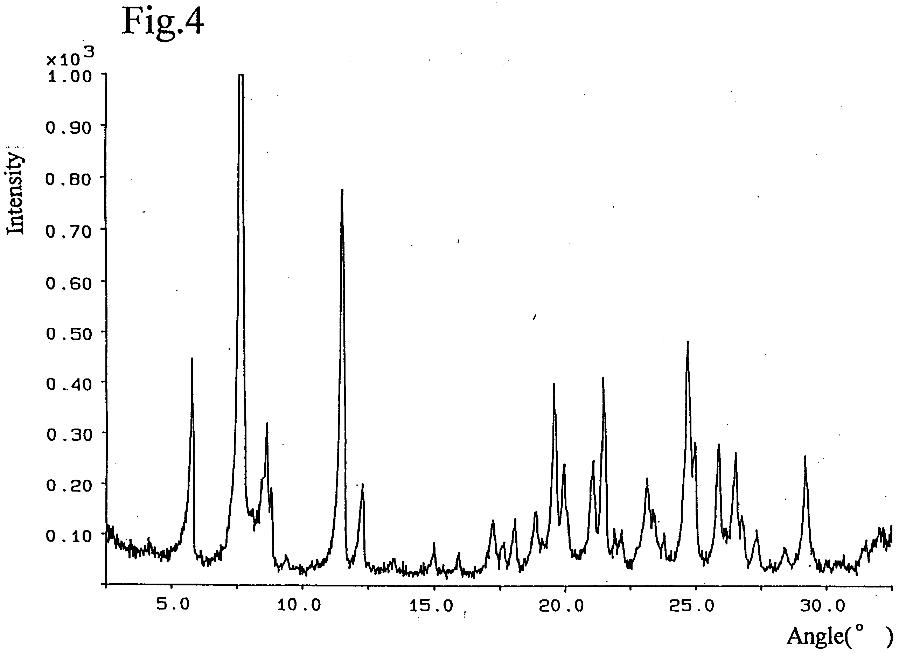

The crystals obtained in Example 6 (all volume) were dried using Chuo Kakoki's vibrating fluidized-bed dryer at an internal humidity setting of 35%-50% and an internal temperature setting of 35°C for 1 day to remove the solvent, followed by drying under reduced pressure to provide 16.0 g or 7β-[2-(2-aminothiazol-4-yl)-2-(Z)-(hydroxyimino)acetamido]-3-[(pyrazol-4-yl)methylthio]-3-cephem-4-carboxylic acid hydrate crystals containing 2.5 molecules of water. The powder X-ray diffraction pattern of the crystals thus obtained is shown in Fig. 4.

### Example 8 (1)

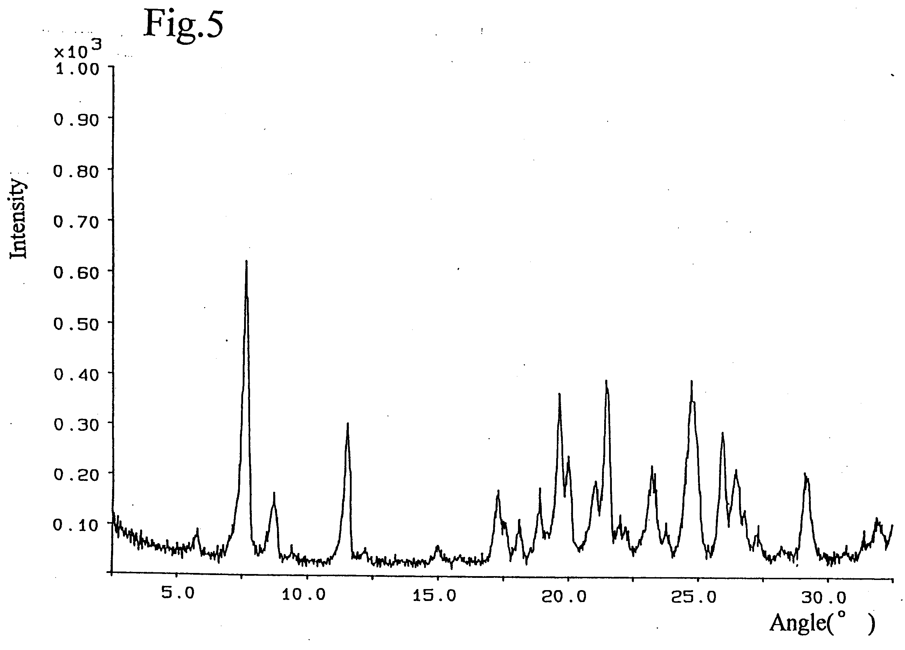

The 7β-[2-(2-aminothiazol-4-yl)-2-(Z)-(-hydroxyimino)acetamido]-3-[(pyrazol-4-yl)methylthio]-3-cephem-4-carboxylic acid (20.0 g) obtained in Preperation 9 was suspended in water (340 ml) and the suspension was cooled to 5-15°C. To this cooled suspension was added sodium hydrogen carbonate (3.6 g) and the mixture was stirred at the same temperature for about 1 hour to prepare a homogeneous solution, which was then filtered. To the filtrate was added isopropyl alcohol (200 ml) and the mixture was warmed to 30°C and adjusted to pH 3.8-4.3 with 1N-hydrochloric acid. The mixture was stirred for 10 hours while its pH was controlled at 3.8-4.3 using 1N-hydrochloric acid. The resulting precipitate was collected by filtration, washed with water (200 ml) and isopropyl alcohol (100 ml), and dried under reduced pressure to provide crystals of 7β-[2-(2-aminothiazol-4-yl)-2-(Z)-(hydroxyimino)acetamido]-3-[(pyrazol-4- yl)methylthio]-3-cephem-4-carboxylic acid containing water (2.7 molecules) and isopropyl alcohol (0.44 molecule). The powder X-ray diffraction pattern of the crystals thus obtained is shown in Fig. 5.

### Example 8 (2)

The above procedure was repeated except that the amount of isopropyl alcohol for crystallization and the drying conditions are varied to provide the following 5 lots of crystals.

**[Table 7]**

| Lot No. | Isopropyl alcohol (%) in the solution | Drying conditions | Water (molecules) | Isopropyl alcohol (molecules) |
|---|---|---|---|---|
| 35524OP | 33 | Drying in high vacuum | 0.68 | 0.36 |
| 35414OP | 33 | Air drying followed by drying under reduced pressure | 2.12 | 0.31 |
| 37914XP | 60 | Drying under reduced pressure | 2.49 | 0.44 |
| 35434OP | 33 | Drying under reduced pressure | 2.72 | 0.44 |
| 35534OP | 33 | Drying under reduced pressure | 2.76 | 0.46 |

### Example 9

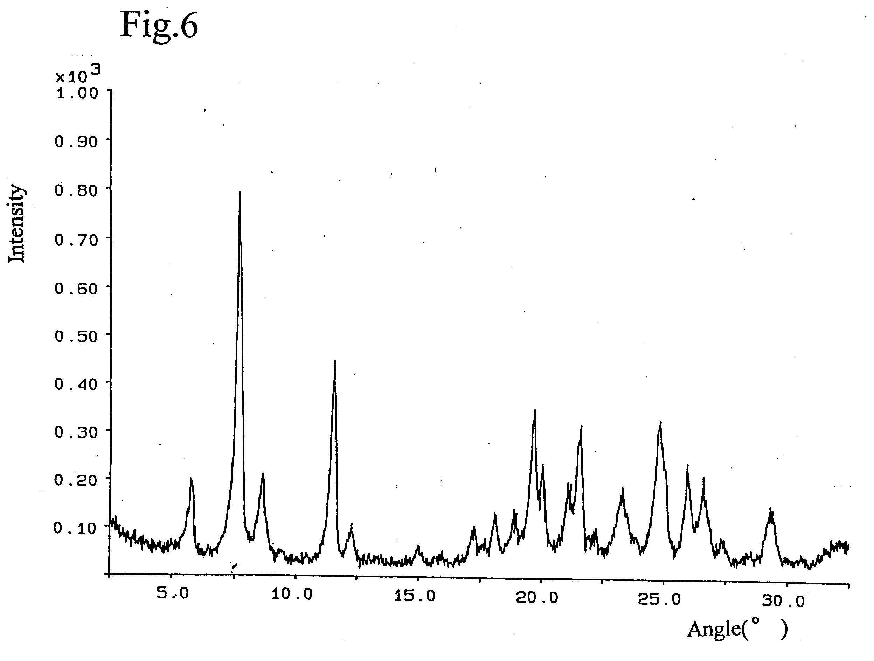

A column of about 200 ml capacity which was equipped with a bottom filter plate was packed with the crystals obtained in Example 8 (1) (all volume). While the external temperature as well as the temperature of the fluid inlet of the column and the internal pressure of the column were controlled at about 40°C and about 200 kg/cm², respectively, carbon dioxide gas is blown through the packing from top to bottom of the column for 1 hour with water being added from a feed port for extractive removal of isopropyl alcohol to provide hydrate crystals of 7β-[2-(2-aminothiazol-4-yl)-2-(Z)-(hydroxyimino)acetamido]-3-[(pyrazol-4-yl)methylthio]-3-cephem-4-carboxylic acid (17.5 g) containing 2.8 molecules of water. The powder X-ray diffraction pattern of the crystals thus obtained is shown in Fig. 6.

### Example 10

The crystals of 7β-[2-(2-aminothiazol-4-yl)-2-(Z)-(hydroxyimino)acetamido]-3-[(pyrazol-4-yl)methylthio]-3-cephem-4-carboxylic acid containing both water and isopropyl alcohol as prepared in the same manner as Example 8 (1) (water 2.4 molecules, isopropyl alcohol 0.57 molecules) were allowed to stand in an atmosphere of isopropyl alcohol vapor for 48 hours to provide crystals of 7β-[2-(2-aminothiazol-4-yl)-2-(Z)-(hydroxyimino)acetamido]-3-[(pyrazol-4-yl)methylthio]-3-cephem-4-carboxylic acid containing 1.8 molecules of water and 1.6 molecules of isopropyl alcohol.

### Example 11

The crystals of 7β-[2-(2-aminothiazol-4-yl)-2-(Z)-(hydroxyimino)acetamido]-3-[(pyrazol-4-yl)methylthio]-3-cephem-4-carboxylic acid containing water (2.3 molecules) as prepared in the same manner as Example 9 were allowed to stand in an atmosphere of isopropyl alcohol vapor for 48 hours to provide crystals of 7β-[2-(2-aminothiazol-4-yl)-2-(Z)-(hydroxyimino)acetamido]-3-[(pyrazol-4-yl)methylthio]-3- cephem-4-carboxylic acid containing 1.9 molecules of water and 0.86 molecule of isopropyl alcohol.

### Example 12

The crystals of 7β-[2-(2-aminothiazol-4-yl)-2-(Z)-(hydroxyimino)acetamido]-3-[(pyrazol-4-yl)methylthio]-3-cephem-4-carboxylic acid containing both water and isopropyl alcohol as prepared in the same manner as Example 8 (1) were allowed to stand in the air at 25°C and relative humidities indicated in Table 8 for 7 days to provide crystals of 7β-[2-(2-aminothiazol-4-yl)-2-(Z)-(hydroxyimino)acetamido]-3-[(pyrazol-4-yl)methylthio]-3-cephem-4-carboxylic acid containing water and isopropyl alcohol in the amounts shown in Table 8.

**[Table 8]**

| Relative humidity | Water of crystallization (molecules) | Isopropyl alcohol of crystallization (molecules) |
|---|---|---|
| Initial | 2.7 | 0.31 |
| Under silica gel desiccation | 2.1 | 0.25 |
| 11% | 2.7 | 0.26 |
| 33% | 3.0 | 0.26 |
| 53% | 3.1 | 0.26 |
| 75% | 3.2 | 0.23 |
| 93% | 3.4 | 0.20 |

## Claims

1. A crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof.

2. The crystal of claim 1, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a solvate.

3. The crystal of claim 2, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a solvate with lower alcohol and water.

4. The crystal of claim 3, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a solvate with ethyl alcohol and water.

5. The crystal of claim 3, the crystal of 7β-[2-(2-aminothiazol-4-yl)-2-(Z)-(hydroxyimino)acetamido]-3-[pyrazol-4-yl) methylthio]3-cephem-4-carboxylic acid or a salt thereof, which is a solvate with isopropyl alcohol and water.

6. The crystal of claim 2, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a solvate with acetone and water.

7. The crystal of claim 2, the crystal of 7β-[2-(2-aminothiazol-4-yl)-2-(Z)-(hydroxyimino)acetamido]-3- [pyrazol-4-yl)methylthio]3-cephem-4-carboxylic acid or a salt thereof, which is a hydrate.

8. The crystal of claim 7, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a hydrate with 2-4 molecules of water.

9. The crystal of claim 2, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a clathrate crystal.

10. The crystal of claim 9, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a clathrate crystal with a solvent.

11. The crystal of claim 10, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a clathrate crystal with a lower alcohol and water.

12. The crystal of claim 11, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a clathrate crystal with ethyl alcohol and water.

13. The crystal of claim 11, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a clathrate crystal with isopropyl alcohol and water.

14. The crystal of claim 11, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a clathrate crystal with acetone and water.

15. The crystal of claim 10, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a clathrate crystal with water.

16. The crystal of claim 11, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which is a clathrate crystal with 2-4 molecules water.

17. The crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof characterized in that which is crystallized from solution containing 7β-[2-(2-aminothiazol-4-yl)-2-(Z)-(hydroxyimino)acetamido]-3-[pyrazol-4-yl)methylthio]3-cephem-4-carboxylic acid or a salt thereof under acidic conditions at cooled temperature, room temperature or elevated temperature.

18. The crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, wherein the solution containing 7β-[2-(2-aminothiazol-4-yl)-2-(Z)-(hydroxyimino)acetamido]-3-[pyrazol-4-yl)methylthio]3-cephem-4-carboxylic acid or a salt thereof contains a lower alcohol and water.

19. A process for producing crystals of 7β-[2-(2-aminothiazol-4-yl)-2-(Z)-(hydroxyimino)acetamido]-3-[pyrazol-4-yl)methylthio]3-cephem-4-carboxylic acid or a salt thereof characterized in that which is crystallized from solution containing 7β-[2-(2-aminothiazol-4-yl)-2-(Z)-(hydroxyimino)acetamido]-3-[pyrazol-4-yl)methylthio]3-cephem-4-carboxylic acid or a salt thereof under acidic conditions at cooled temperature, room temperature or elevated temperature.

20. The process for producing crystals of 7β-[2-(2-aminothiazol-4-yl)-2-(Z)-(hydroxyimino)acetamido]-3-[pyrazol-4-yl)methylthio]3-cephem-4-carboxylic acid or a salt thereof according to claim 19 wherein the solution containing 7β-[2-(2-aminothiazol-4-yl)-2-(Z)-(hydroxyimino)acetamido]-3-[pyrazol-4-yl)methylthio]3-cephem-4-carboxylic acid or a salt thereof contains a lower alcohol and water.

21. A crystal of 7β-[2-(2-aminothiazol-4-yl)-2-(Z)-(hydroxyimino)acetamido]-3-[pyrazol-4-yl)methylthio]3-cephem-4-carboxylic acid which shows peaks at the following diffraction angles in its a powder x-ray diffraction pattern: ca. 7.5°, ca. 11.3°, ca. 19.5°, ca.21.3°, ca. 24.5°, ca. 25.9°, ca. 29.0°.

22. A process for producing 7β-[2-(2-aminothiazol-4-yl)-2-(Z)-(hydroxyimino)acetamido]-3-[pyrazol-4-yl)methylthio]3-cephem-4-carboxylic acid or a salt thereof hydrate crystals characterized in that 7β-[2-(2-aminothiazol-4-yl)-2-(Z)-(hydroxyimino)acetamido]-3-[pyrazol-4-yl)methylthio]3-cephem-4-carboxylic acid or a salt thereof solvate crystals containing water and organic solvent are subjected to treatment for removal of the organic solvent.

23. The process for producing 7β-[2-(2-aminothiazol-4-yl)-2-(Z)-(hydroxyimino)acetamido]-3-[pyrazol-4-yl)methylthio]3-cephem-4-carboxylic acid or a salt thereof hydrate crystals as claimed in Claim 22 wherein said treatment for removal of the organic solvent is supercritical gas extraction.

24. The process for producing 7β-[2-(2-aminothiazol-4-yl)-2-(Z)-(hydroxyimino)acetamido]-3-[pyrazol-4-yl)methylthio]3-cephem-4-carboxylic acid or a salt thereof hydrate crystals as claimed in Claim 22 wherein said treatment for removal of the organic solvent is air current drying using moisture-laden air.

25. The crystal of claim 1, the crystal of 7β-[2-(2-aminothiazol-4-yl)-2-(Z)-(hydroxyimino)acetamido]-3-[pyrazol-4-yl)methylthio]3-cephem-4-carboxylic acid or a salt thereof, which contains water and/or solvent.
